Europäisches Patentamt

European Patent Office

Office européen des brevets

(11) Publication number: **0 496 138 A1**

## EUROPEAN PATENT APPLICATION

(21) Application number: **91300195.4**

(22) Date of filing: **11.01.91**

(51) Int. Cl.5: **A61K 31/46**, A61K 31/135, A61K 9/72, A61K 9/12

(30) Priority: **17.01.91 GB 9001019**

(43) Date of publication of application:
**29.07.92 Bulletin 92/31**

(84) Designated Contracting States:
**AT BE CH DE DK ES FR GB GR IT LI LU NL SE**

(71) Applicant: **Euroceltique SA**
**122 Boulevard de la Petrusse**
**Luxembourg(LU)**

(72) Inventor: **Leslie, Stewart Thomas**
**4 Babraham Road**
**Cambridge(GB)**
Inventor: **Malkowska, Sandra Therese**
**Antoinette**
**21 Broadway, Wilburton**
**Ely, Cambridge(GB)**
Inventor: **Miller, Ronald Brown**
**'Résidence du Soleil',Schützenmattstrasse**
**40**
**CH-4051 Basel(CH)**

(74) Representative: **Lamb, John Baxter et al**
**MARKS & CLERK 57-60 Lincoln's Inn Fields**
**London WC2A 3LS(GB)**

(54) Atropine-salbutamol aerosol.

(57) A pressurised, metered-dose aerosol contains an orally inhalable, pharmaceutical composition comprising a mixture of salbutamol sulphate, at least 90% (by wt) of which has a particle size of less than 10 microns, atropine methonitrate, at least 90% (by wt) of which has a particle size of less than 10 microns, and at least one of a surfactant and a wetting agent suspended in an aerosol propellant, wherein each actuation of the aerosol valve delivers salbutamol sulphate equivalent to from 50mcg to 200mcg of salbutamol and atropine methonitrate equivalent to from 50mcg to 300mcg of atropine.

EP 0 496 138 A1

The present invention relates to a pharmaceutical aerosol and to a pharmaceutical composition for use in the aerosol. In particular it relates to an aerosol containing a pharmaceutical composition comprising a mixture of salbutamol and atropine.

The administration of a combination of salbutamol and atropine methonitrate by wet nebulisation is described in R J Pierce et al, Thorax, 34, 45-50 (1979) and by I D Starke et al in Respiration, 43, 51-56 (1982).

We have now surprisingly found that the delivery of a combination of a low dose of salbutamol sulphate and a low dose of atropine methonitrate from a pressurised aerosol gives enhanced bronchodilation.

According to a first aspect the present invention therefore provides a pressurised, metered-dose aerosol containing an orally inhalable, pharmaceutical composition comprising a mixture of salbutamol sulphate, at least 90% (by wt) of which has a particle size of less than 10 microns, atropine methonitrate, at least 90% (by wt) of which has a particle size of less than 10 microns, and at least one of a surfactant and a wetting agent suspended in an aerosol propellant wherein each actuation of the aerosol valve delivers salbutamol sulphate equivalent to from 50mcg to 200mcg of salbutamol and atropine methonitrate equivalent to from 50mcg to 300mcg of atropine.

Preferably 90% (by wt) of the droplets sprayed from the aerosol have a droplet size of 10 microns or less, especially between 0.5 and 10 microns.

According to a second aspect of the present invention, there is provided an orally inhalable, pharmaceutical composition comprising a mixture of salbutamol sulphate, at least 90% (by wt) of which has a particle size of less than 10 microns, atropine methonitrate, at least 90% (by wt) of which has a particle size of less than 10 microns, and at least one of a surfactant and a wetting agent suspended in an aerosol propellant. According to a further aspect the invention provides a process for preparing an orally inhalable pharmaceutical composition which comprises mixing salbutamol sulphate, at least 90% by weight of which has a particle size of less than 10 microns, atropine methonitrate, at least 90% by weight of which has a particle size of less than 10 microns, as least one of a surfactant and a wetting agent and an aerosol propellant and filling the mixture into an aerosol container provided with a metering valve such that each actuation of the aerosol valve delivers salbutamol sulphate equivalent to from 50mcg to 200mcg of salbutamol and atropine methonitrate equivalent to from 50mcg to 300mcg of atropine.

In a preferred embodiment of the present aerosol/composition, the particle size of at least 90% (by wt) of the salbutamol sulphate is preferably between 0.5 and 10 microns. Similarly, at least 90% (by wt) of the atropine methonitrate preferably has a particle size between 0.5 and 10 microns.

In a particularly preferred embodiment of the present aerosol the particle size of at least 80% (by wt) of the salbutamol sulphate is below 5 microns, especially between 0.5 and 5 microns. Similarly, at least 80% (by wt) of the atropine methonitrate preferably has a particle size below 5 microns, especially between 0.5 and 5 microns.

The surfactant/wetting agent must be compatible with salbutamol sulphate, atropine methonitrate and the aerosol propellant. It should be non-toxic, biodegradable and minimally irritating to the respiratory airways. Preferred surfactants/wetting agents are also odourless, tasteless and colourless.

Suitable surfactants/wetting agents for use according to the present invention are those conventionally known in the art and include, for example, oleic acid, oleyl alcohol, lecithin, sorbitan monooleate (trade name Span 80), sorbitan trioleate (trade name Span 85), sorbitan monolaurate (trade name Span 20), polyoxyethylene (20) sorbitan monolaurate (trade name Tween 20), polyoxyethylene (20) sorbitan monooleate (trade name Tween 80), oleyl polyoxyethylene ether (trade name Brij 92), stearyl polyoxyethylene (trade name Brij 72) and oils such as olive oil, cotton seed oil and sunflower seed oil. Sorbitan trioleate is particularly preferred. The surfactant/wetting agent is conveniently present in an amount of from 0.005% to 6% by weight, preferably from 0.01% to 5% by weight, most particularly from 0.1% to 2% by weight.

A number of compressed and liquefied gases may be used as aerosol propellants. These include nitrogen, nitrous oxide, carbon dioxide, propane and the butanes. Preferably, however, the propellant comprises one or more halogenated hydrocarbons, especially chlorofluorocarbons, such as dichlorodifluoromethane, dichlorotetrafluoroethane and trichlorofluoromethane; hydrofluorocarbons such as hydrofluorocarbon 125, 134a, 143a, 152a; and hydrochlorofluorocarbons such as HCFC 22, 123, 124, 141b and 142b. In a preferred embodiment of the present aerosol, the propellant is a mixture of dichlorodifluoromethane and trichlorofluoromethane, conveniently in a ratio of dichlorodifluoromethane to trichlorofluoromethane of from 80:20 to 60:40.

Each actuation of the present aerosol valve must deliver a predetermined dose in the range 50 to 200mcg of salbutamol (as salbutamol sulphate) and a predetermined dose in the range 50 to 300mcg of atropine (as atropine methonitrate). In preferred embodiments of the present aerosol, each actuation will deliver 75 to 175mcg, especially 100 to 150mcg, of salbutamol (as salbutamol sulphate) and 50 to 275mcg, especially 100 to 250mcg, of atropine (as atropine methonitrate).

In a particularly preferred embodiment each actuation delivers salbutamol sulphate equivalent to 100mcg salbutamol and atropine methonitrate equivalent to 50mcg atropine.

In another particularly preferred embodiment each actuation delivers salbutamol sulphate equivalent to 100mcg salbutamol and atropine methonitrate equivalent to 150mcg atropine.

The active ingredients for use in the present invention may be micronised to the desired particle size range by conventional techniques for example using a ball mill or by air jet milling. The pharmaceutical composition for use according to the invention may be prepared by mixing the ingredients together, for example by dispersing the active ingredients and surfactant in the propellant using a mixer. The composition may be filled into a conventional can and the can valve crimped on. Other propellants may be filled into the can through the valve.

In order that the invention may be well understood the following examples are given by way of illustration only.

Method of Particle Size Determination

The particle size of the active ingredients and droplet size of the finished product are determined by laser light diffraction based on the Fraunhoffer diffraction theory. The particle size correlates with the angle through which the particle defracts light; small particles diffract light at high angles and larger particles defract it at smaller angles.

Each active ingredient is suspended uniformly in a solvent in which the drug is insoluble, or which has previously been saturated with the drug. The drug suspension is placed in the laser and the diffraction of light recorded and correlated to the particle size distribution of the drug.

The droplet size of the finished product is measured by actuation of the aerosol across the laser beam. The diffraction of the light is detected and correlated with the particle size distribution of the droplets.

In the following examples, 90% by weight the active ingredients have a particle size of less than 10 microns as determined by the method described above.

Example 1

Micronised atropine methonitrate (0.07g), micronised salbutamol sulphate (0.132g), sorbitan trioleate (0.135g) and trichlorofluoromethane (17.863g) were mixed until a uniform dispersion was obtained. 4.55g of this mixture was then filled into an aerosol can. After a valve was attached to the can, dichlorodifluoromethane (11.70g) was added to the can, using pressure filling equipment.

Each actuation of the can delivered the following composition,

|  | mg/actuation |
| --- | --- |
| Atropine as atropine methonitrate (micronised) | 0.055 |
| Salbutamol as salbutamol sulphate (micronised) | 0.110 |
| Sorbitan trioleate | 0.135 |
| Trichlorofluoromethane | 17.863 |
| Dichlorodifluoromethane | 46.800 |

A 10% overage is added to counteract potential losses on manufacture and/or stability.

Example 2

Micronised atropine methonitrate (0.0633g), micronised salbutamol sulphate (0.1205g), sorbitan trioleate (0.135g) and trichlorofluoromethane (17.7212) were mixed until a uniform dispersion was obtained. 4.51g of this mixture was then filled into an aerosol can. After a valve was attached to the can, dichlorodifluoromethane (11.70g) was added to the can using pressure filling equipment.

Each actuation of the can delivered the following composition:

EP 0 496 138 A1

|  | mg/actuation |
|---|---|
| Atropine as atropine methonitrate (micronised) | 0.050 |
| Salbutamol as salbutamol sulphate (micronised) | 0.100 |
| Sorbitan trioleate | 0.135 |
| Trichlorofluoromethane | 17.721 |
| Dichlorodifluoromethane | 46.800 |

Example 3

The process of Example 2 was repeated except that the amount of atropine methonitrate employed was increased to 0.1266g, and the trichlorofluoromethane reduced to 17.6579g.

Example 4

The process of Example 2 was repeated except that the amount of atropine methonitrate employed was increased to 0.1899g, and the trichlorofluoromethane reduced to 17.5946g.

Example 5

The process of Example 2 was repeated except that the amount of salbutamol sulphate employed was increased to 0.1808g, and the trichlorofluoromethane reduced to 17.6610g.

Example 6

The process of Example 2 was repeated using atropine methonitrate (0.0475g), salbutamol sulphate (0.0303g), sorbitan trioleate (0.163g), trichlorofluoromethane (4.28g) and dichlorodifluoromethane (11.7g).
Each actuation of the can delivered the following composition.

|  | mg/actuation |
|---|---|
| Atropine as atropine methonitrate (micronised) | 0.190 |
| Salbutamol as salbutamol sulphate (micronised) | 0.121 |
| Sorbitan trioleate | 0.650 |
| Trichlorofluoromethane | 17.1 |
| Dichlorodifluoromethane | 46.8 |

Example 7

The process of Example 6 was repeated except that sorbitan trioleate was replaced with soya lecithin.

CLINICAL STUDIES

Sixteen patients with chronic, stable asthma were recruited. The study was to a four-arm, three point crossover, prospective randomised Latin Square, placebo controlled design. Each treatment arm was of 3 days. On the first day of each arm the patient received placebo inhaler, on the second day one actuation twice daily of the test inhaler and the third day two actuations of the test inhaler twice daily. The inhalers tested were:-
  - Salbutamol sulphate - Atropine methonitrate combination (Example 1 above)
  - Salbutamol 100mcg per actuation
  - Atropine methonitrate 50mcg per actuation
  - Placebo
$FEV_1$, PEFR and FVC were measured before the morning dose on each day and thereafter for twelve hours. The results showed that in terms of maximal increase $FEV_1$ the combination was significantly different from placebo ($p<0.01$) and salbutamol was significantly different from placebo ($p<0.05$).

4

Atropine methonitrate alone was not significantly different from placebo or the other two treatments.

The mean curves indicated that the bronchodilator effect of the combination was more long lasting than that due to salbutamol.

**Claims**

1. A pressurised, metered-dose aerosol containing an orally inhalable, pharmaceutical composition comprising a mixture of salbutamol sulphate, at least 90% (by wt) of which has a particle size of less than 10 microns, atropine methonitrate, at least 90% (by wt) of which has a particle size of less than 10 microns, and at least one of a surfactant and a wetting agent suspended in an aerosol propellant, wherein each actuation of the aerosol valve delivers salbutamol sulphate equivalent to from 50mcg to 200mcg of salbutamol and atropine methonitrate equivalent to from 50mcg to 300mcg of atropine.

2. An aerosol according to Claim 1 wherein at least 80% by weight of the salbutamol sulphate has a particle size between 0.5 and 5 microns.

3. An aerosol according to Claim 1 wherein at least 80% by weight of the atropine methonitrate has a particle size between 0.5 and 5 microns.

4. An aerosol according to any of Claims 1 to 3 wherein each actuation through the valve delivers salbutamol sulphate equivalent to from 75 to 175mcg salbutamol and atropine methonitrate equivalent to from 50 to 275mcg atropine.

5. An aerosol according to claim 4 wherein each actuation through the valve delivers salbutamol sulphate equivalent to from 100 to 150mcg salbutamol and atropine methonitrate equivalent to from 100 to 250mcg atropine.

6. An aerosol according to claim 5 wherein each actuation through the valve delivers salbutamol sulphate equivalent to 100mcg salbutamol and atropine methonitrate equivalent to 150mcg atropine.

7. An aerosol according to any of claims 1 to 6 wherein the surfactant is sorbitan trioleate.

8. An aerosol according to any of claims 1 to 7 wherein the propellant comprises a mixture of dichlorodifluoromethane and trichlorofluoromethane.

9. An orally inhalable, pharmaceutical composition comprising a mixture of salbutamol sulphate, at least 90% (by wt) of which has a particle size of less than 10 microns, atropine methonitrate, at least 90% (by wt) of which has a particle size of less than 10 microns, and at least one of a surfactant and a wetting agent suspended in an aerosol propellant.

**Claims for the following Contracting States : ES, GR**

1. A process for preparing an orally inhalable, pharmaceutical composition which comprises mixing salbutamol sulphate, at least 90% (by wt) of which has a particle size of less than 10 microns, atropine methonitrate, at least 90% (by wt) of which has a particle size of less than 10 microns, and at least one of a surfactant and a wetting agent and an aerosol propellant, and filling the mixture into an aerosol container provided with a metering valve such that each actuation of the aerosol valve delivers salbutamol sulphate equivalent to from 50mcg to 200mcg of salbutamol and atropine methonitrate equivalent to from 50mcg to 300mcg of atropine.

2. A process according to Claim 1 wherein at least 80% by weight of the salbutamol sulphate has a particle size between 0.5 and 5 microns.

3. A process according to Claim 1 wherein at least 80% by weight of the atropine methonitrate has a particle size between 0.5 and 5 microns.

4. A process according to any of Claims 1 to 3 wherein each actuation through the valve delivers salbutamol sulphate equivalent to from 75 to 175mcg salbutamol and atropine methonitrate equivalent to from 50 to 275mcg atropine.

5. A process according to claim 4 wherein each actuation through the valve delivers salbutamol sulphate equivalent to from 100 to 150mcg salbutamol and atropine methonitrate equivalent to from 100 to 250mcg atropine.

6. An process according to claim 5 wherein each actuation through the valve delivers salbutamol sulphate equivalent to 100mcg salbutamol and atropine methonitrate equivalent to 150mcg atropine.

7. A process according to any of claims 1 to 6 wherein the surfactant is sorbitan trioleate.

8. A process according to any of claims 1 to 7 wherein the propellant comprises a mixture of dichlorodifluoromethane and trichlorofluoromethane.

## DOCUMENTS CONSIDERED TO BE RELEVANT

EP 91300195.4

| Category | Citation of document with indication, where appropriate, of relevant passages | Relevant to claim | CLASSIFICATION OF THE APPLICATION (Int. Cl.5) |
|---|---|---|---|
| D,A | THORAX, vol. 34, 1979, London<br>R.J. PIERCE et al. "A comparative study of atropine methonitrate, salbutamol, and their combination in airways obstruction"<br>pages 45-50<br>   * Abstract; page 46, right column, first paragraph; page 47, right column, second paragraph, table 3; page 49, left column, first, second paragraph, right column, second paragraph * | 1,4,5,<br>6,9 | A 61 K 31/46<br>A 61 K 31/135<br>A 61 K 9/72<br>A 61 K 9/12 |
| D,A | RESPIRATION, vol. 43, 1982, Basel<br>I.D. STARKE et al. "Atropine Methonitrate and Salbutamol in Chronic Airways Obstruction: Peak Effect and Duration of Action"<br>pages 51-56<br>   * Abstract; page 52, left column, second paragraph; right column, figure 1; page 53, figures 2,3; page 54, table 1, discursion * | 1,9 | |
| A | WO - A1 - 86/06 959<br>(LIPOSOME TECHNOLOGY, INC.)<br>   * Claims 1,2,6-9,15; page 19, lines 25-32 * | 1-3,<br>7-9 | TECHNICAL FIELDS SEARCHED (Int. Cl.5)<br><br>A 61 K 31/00<br>A 61 K 9/00 |
| A | CHEMICAL ABSTRACTS, vol. 77, no. 25, December 18, 1972, Columbus, Ohio, USA<br>P.GAYRARD et al.<br>" Effects of various bronchodilator agents on airway con- | 1,4-6,<br>9 | |

The present search report has been drawn up for all claims

| Place of search | Date of completion of the search | Examiner |
|---|---|---|
| VIENNA | 05-02-1991 | MAZZUCCO |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | ductance in asthma, after beta-adrenergic blockade" page 36, column 2, abstract-No. 160 268m<br>   & Bull. Physio-Pathol. Resp. 1972,8(3),625-41<br>   --<br>& Bull. Physio-Pathol. Resp. 1972, 8(3), 625-41<br>   ---- | | |
| | | | TECHNICAL FIELDS SEARCHED (Int. Cl.5) |

The present search report has been drawn up for all claims

| Place of search | Date of completion of the search | Examiner |
|---|---|---|
| VIENNA | 05-02-1991 | MAZZUCCO |